# EUROPEAN PATENT APPLICATION

(11) **EP 0 872 254 A2**
(43) Date of publication of application: **21.10.1998**
(21) Application number: 98302311.0
(22) Date of filing: 26.03.1998
(51) Int. Cl.: A61M 16/12

(54) **Nitric oxide monitoring system**

(30) Priority: 18.04.1997 US 884218
(71) Applicant: OHMEDA INC., Liberty Corner, New Jersey 07938-0804 (US)
(72) Inventor: Wobermin, Jim, Arvada, Colorado 80004 (US); Gull, Kathleen A. Bergeron, Sunnyvale, California 97087 (US); Gonzales, Charles, Westminster, Colorado 80234 (US)
(74) Representative: Hedley, Nicholas James Matthew

(57) **Abstract**

The inhaled nitric oxide system (9) includes a respiratory circuit (12) for delivering gases to a patient (14), a source (16) of gas containing NO, an NO monitoring and delivery subsystem (18) and a gas scavenger (20). The NO monitoring subsystem (18) includes electrochemical cells located on a side stream flow line for monitoring NO delivery. In one embodiment, the monitoring subsystem includes, in serial sequence on the side stream flow line, an NO₂ cell, an NO cell and an O₂ cell. The cells are housed in sealed chambers upstream from a flow pump, and thus operate at subambient pressures. The system (10) provides accurate and continuous monitoring of inhaled nitric oxide administration using electrochemical cell technology, and provides fast response time with reduced cross-cell interference.

## Description

The present invention relates generally to the use of inhaled gas for anesthetic and/or therapeutic purposes and, in particular, to monitoring the administration of gas to patients in connection with inhaled nitric oxide (INO) therapy.

The effectiveness of INO therapy has been investigated or established in connection with a variety of medical conditions. In particular, research has shown that INO therapy has a vasodilatory effect and may increase oxygen saturation for newborns having persistent pulmonary hypertension. INO therapy is also being investigated for treating adult and infant Respiratory Distress Syndrome and may find further applications. In such cases, INO therapy may be administered in intensive care units, newborn intensive care units, operating rooms and other settings.

In INO therapy, nitric oxide (NO) is administered to the patient together with other respiratory gases. The NO is supplied from a remote or on-site source of gas containing NO. For example, the source may be an on-site pressurized gas canister containing a mixture of NO and nitrogen (N₂) or other inert gases. The gas is typically delivered at a pressure of 2000 psi or more with a concentration of NO in the range of 400-2000 ppm. This gas is mixed, in a closely controlled manner, with the respiratory gases just prior to inhalation by the patient. The composition of respiratory gases may vary depending on a number of factors but will include a concentration of oxygen (O₂) sufficient for patient respiration.

The administration of INO therapy is complicated due to interaction of the component gases. In particular, the NO containing gas (e.g., an N₂/NO mixture) interacts with the O₂ containing respiratory gas to produce oxides of nitrogen including NO and nitrogen dioxide (NO₂). Such interactions cause the concentration of NO and O₂ to vary within the patient inspiration line. Moreover, NO₂, if not properly monitored and controlled, could be harmful to the patient in large concentrations. Consequently, it is desirable to monitor NO, NO₂ and O₂ in the patient inspiration line to ensure proper administration of respiratory and therapeutic gases as well as to ensure that patient health is fully protected.

Experience with monitoring INO therapy is somewhat limited due to the relatively recent development and acceptance of such therapy as well as delays in gaining approval for such therapy in certain jurisdictions. In some cases, INO therapy has been monitored or controlled through careful administration of gases by highly skilled personnel and/or by periodically extracting gas samples for separate analysis. Recently introduced NO delivery systems have included dedicated NO, NO₂ and O₂ sensors. There is a continuing need, however, for systems that provide continuous and accurate INO therapy monitoring.

The present invention allows for continuous monitoring of INO therapy and reduces or substantially eliminates errors or uncertainties that might otherwise result due to varying pressures and flow rates associated with patient breathing and pump cycling. In addition, the invention reduces interference errors that can occur due to operation of multiple sensors to detect multiple components of a given gas sample. These advantages can be achieved without unduly increasing NO/O₂ reaction times in the patient inspiration line.

According to one aspect of the present invention, a side stream monitoring assembly is employed to monitor INO therapy. The monitoring assembly is used in conjunction with an NO delivery assembly including a supply of gas containing NO, a patient interface such as a mouthpiece, intratracheal tube or mask, and an inspiration flow line for delivering a first portion of the NO containing gas from the source to the patient interface. The NO gas will normally mix with separately supplied respiratory gases in the inspiration flow line. The side stream monitoring assembly includes a side stream flow line for receiving a second portion of the NO containing gas (e.g., together with respiratory gases) diverted from the inspiration flow line and an electrochemical cell associated with the side stream flow line for use in sensing the amount of nitric oxide in the diverted gas portion in the side stream flow line.

This side stream structure has particular advantages with respect to use of electrochemical cells. Such cells have been developed for use in industrial applications and generally have slow response times in relation to respiratory dynamics. They typically operate most effectively when employed in connection with substantially constant gas flows and pressures. The medical environment, by contrast, is characterized by widely varying gas flows and pressures such as associated with the breathing cycle and pump dynamics. Moreover, the diffusion barrier of electrochemical cells can become clogged due to liquids and other matter as may be present in an INO therapy system. The side stream structure of the present invention allows for continuous extraction of a portion of the gas into an environment where the pressure and flow fluctuations of the gas flow across the cells can be controlled without affecting the dynamics of the respiratory circuit, and liquids or other matter can be removed before reaching the cells. Moreover, the side stream structure accommodates sensors that may be too large or heavy to mount on neonatal or other breathing apparatus.

Preferably, the side stream sensing assembly includes sensors for measuring the amounts of NO₂ and O₂ in the side stream, in addition to the NO sensor. The NO₂ and O₂ sensors may also be electrochemical cells. The NO and NO₂ sensors can be disposed in parallel flow lines or in series on a single flow line. In one embodiment, the NO and NO₂ cells are disposed in parallel to reduce interference resulting from chemical interaction at the diffusion barriers of the electrochemical cells.

According to another aspect of the present invention, an INO monitoring system includes an NO sensor that is disposed upstream from a pump for drawing gas through a flow line. More particularly, the system includes a supply of gas containing nitric oxide, a flow line for use in delivering gas from the source, a pump for drawing gas into the flow line, and an NO sensor such as an electrochemical cell disposed between the supply and the pump. In one embodiment, the sensor is mounted in a side stream flow line connected to a patient inspiration line. An O₂ sensor and an NO₂ sensor may also be included with the NO sensor upstream from the pump. It has been found that, if the NO sensor is located downstream from the pump, the sensor will experience pump generated pressure waves including pressures of at least about equal to the ambient pressure. These waves can interfere with concentration measurements. Additionally, an upstream pump may undesirably mix the sample gas prior to the concentration measurement. By locating the sensor upstream from the pump, pressure fluctuations can be easily controlled and undesirable mixing is avoided.

According to a further aspect of the invention, the NO sensing chamber is sealed from the ambient environment. The associated INO therapy system includes an NO sensor disposed in a housing, a sample gas line for delivering gas into the housing to the sensor, and a seal for sealing the housing so as to maintain a pressure differential between the interior of the housing and the ambient environment. In this manner, the pressure within the housing can be caused to relate more closely to the dynamics of a patient inspiration line. Preferably, the housing is maintained at a subambient pressure, (e.g., due to positioning of the chamber upstream from a vacuum pump). The sealed housing environment allows for accurate concentration measurements. In particular, in the case of an electrochemical cell where the cell is placed within a sealed housing, the partial pressure drop across the cell's diffusion barrier will be due to gas concentration and not due to a pressure difference between the sample gas pressure and ambient pressure.

According to a still further aspect of the present invention, a sequence of the system sensors is selected to reduce sensor interference as in a serial sensing application. It has been found that the ordering of NO and NO₂ cells can influence measurement performance. The associated INO therapy system includes a supply of gas containing NO, a flow line for the gas, an NO sensor disposed in the flow line, and an NO₂ sensor disposed in the flow line between the source and the NO sensor. That is, the NO and NO₂ sensors are arranged in series with the NO₂ sensor located upstream from the NO sensor. This ordering is believed to be beneficial, particularly in the case of electrochemical cell sensing, because NO may be generated during the time/process of sensing NO₂. In one embodiment, NO₂, NO and O₂ sensors are arranged in that order. The sample gas is routed across the sensors in series by narrow flow lines and other flow line defining structure so as to reduce dead space, thereby enhancing cell response time and accuracy.

The invention thus allows for continuous and accurate INO therapy monitoring. Such monitoring can be conducted using electrochemical cells which have previously been used in industrial environments under more constant flow conditions. The invention provides short response times and facilitates NO, NO₂ and O₂ sensing with reduced sensor interference.

For a more complete understanding of the present invention and further advantages thereof, reference is now made to the following detailed description, taken in conjunction with the accompanying diagrammatic drawings, in which:
Fig. 1 is a block diagram of an INO therapy system according to the present invention;
Fig. 2 is a block diagram illustrating an embodiment of a gas monitoring system according to the present invention employing a parallel NO/NO₂ sensor arrangement;
Fig. 3 is a block diagram showing the sample gas flow across NO₂, NO and O₂ sensors arranged in series;
Fig. 4 is an exploded perspective view of an electrochemical cell assembly such as may be employed for the NO and NO₂ sensor units in accordance with the present invention;
Fig. 5 is an exploded side view, partially in cross-section, of the electrochemical cell assembly of Fig. 4;
Figs. 6A - 6C are top, side and bottom views, respectively, of a monitoring unit base in accordance with the present invention; and
Figs. 7A - 7C show top, side and bottom views, respectively, of a cell cap for use in conjunction with the base of Figs. 6A - 6C.

In the following description, the invention is set forth with respect to various embodiments of an INO system with side stream gas concentration monitoring employing electrochemical sensors. However, it will be appreciated that certain aspects of the invention are more broadly applicable to medical gas delivery or monitoring systems employing various types of concentration measurement devices.

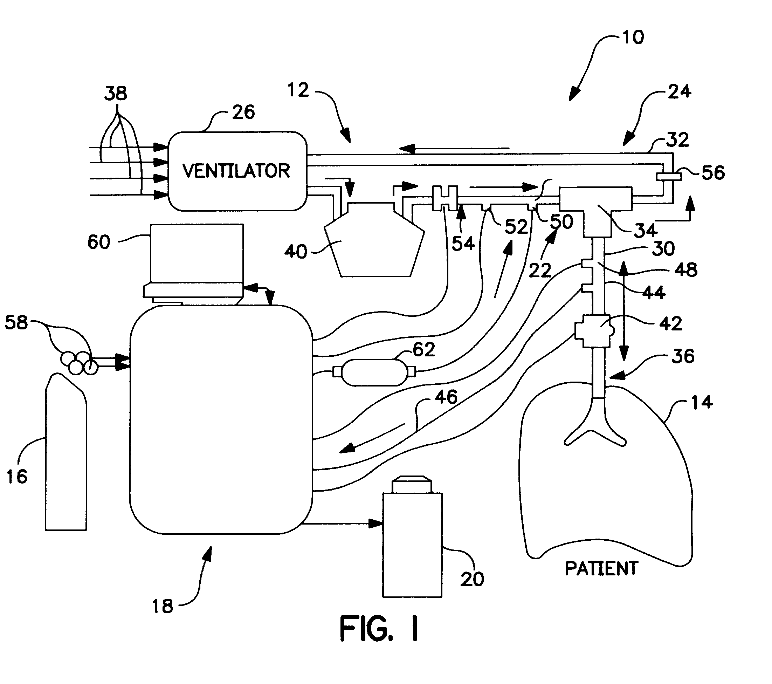

Referring to Fig. 1, an INO system 10 according to the present invention is shown in block diagram form. Generally, the system 10 includes a respiratory circuit 12 for delivering gases to a patient 14, a source 16 of gas containing NO, an NO monitoring and delivery subsystem 18, and a gas scavenger 20. Each of these component parts of the system 10 is described in turn below.

The respiratory circuit 12 includes an inspiratory limb 22 and an expiratory limb 24, each extending between conventional ventilator 26 and the patient 14. The illustrated inspiratory limb 22 is composed of inspiratory line 28 and patient tube 30. The expiratory limb 24 is composed of the patient tube 30 and expiratory line 32. It will thus be appreciated that the patient tube 30 accommodates bi-directional flow and forms a portion of both the inspiratory 22 and expiratory 24 limbs. The patient tube 30, inspiratory line 28 and expiratory line 32 are joined by a conventional we valve 34 that operates in synchronization with the patient's breathing to control flow through the inspiratory 22 and expiratory 24 limbs. Proximate to the patient 14, the patient tube 30 terminates in a conventional mask or endotracheal breathing tube 36 for interfacing with the patient's respiratory system.

The ventilator 26 is conventionally used to deliver respiratory and/or anesthetic gases to the patient 14, depending on the particular application. In this regard, the ventilator 26 may receive inputs of air, O₂, N₂O and anesthetic agents (e.g., Isoflurane, Enflurane, Halothane, Desflurane and Sevoflurane), as generally indicated by arrows 38, from appropriate sources. Such gases or mixtures, generally referred to as respiratory gases, pass from the ventilator 26 to a heated humidifier 40 that conditions the respiratory gases for inhalation by the patient 14.

Also mounted on the inspiratory limb 22 of the illustrated embodiment are six NO subsystem interfaces. These are, in reverse flow sequence: a flow sensor 42, such as a Hamilton Medical Differential Pressure Flow Sensor, for bidirectional gas flow measurement at the patient 14; a sample gas port 44, located at or next to the wye valve 34, for diverting a portion of the flow from the patient tube 30 into a side stream 46 to NO subsystem 18 (a temperature sensor may also be included at this location to sense the temperature of gas inhaled and/or exhaled by the patient 14); a pressure sensor 48 for sensing the proximal patient airway pressure; a port 50 for injecting an NO containing gas (e.g., NO/N₂) into the inspiratory limb 22; a sensor unit 52 for distal airway pressure and temperature measurement of the heated, saturated respiratory gases delivered by the ventilator 26; and a flow measurement sensor 54, such as a Turbine Vane Transducer, for respiratory gas flow measurement. The functions of these components will be more fully understood upon consideration of the description below. It will be appreciated that certain of these elements can be relocated or eliminated in accordance with the present invention.

The expiratory limb 24 includes an expiratory valve 56 for ensuring one-way flow through the expiratory line 32 of gases expired by the patient 14. The expired gases may include carbon dioxide (CO₂), O₂, N₂, nitrogen trioxide (N₂O₃) and anesthetic agents. The expiratory line 32 delivers the expired gases to the ventilator 26. From the ventilator 26 the expired gases may be scrubbed and scavenged prior to venting.

The source 16 of NO-containing gas can be a gas pipeline, as provided in some operating rooms and intensive care units, or a gas canister(s) as shown. Generally, such gas canisters of NO are delivered in a pressurized state and a typical pressure is on the order of about 2000 psi with a concentration of NO on the order of about 400-2000 ppm. The remainder of the gas in the canister may be N₂ or another preferably inert gas. Pressure regulators 58 are employed in the illustrated embodiment to reduce the pressure of the NO-containing gas to acceptable levels for operation of the system 10, e.g., to about 40 psi or lower. A solenoid shutoff valve (not shown) may also be employed in conjunction with the source 16 to provide ON/OFF capability independent of mechanical valving. A separate supply of preferably inert gas such as N₂ may be employed to reduce the concentration of NO as necessary.

The NO subsystem 18 performs a number of functions including: monitoring respiration circuit parameters; receiving the side stream 46 and measuring concentrations of NO₂, NO and O₂; determining the concentrations of NO₂, NO and O₂ delivered to the patient 14 based on the measured concentrations; and controllably delivering NO-containing gas to the inspiratory limb 22 in amounts appropriate for achieving the desired therapeutic effects. The desired concentration of delivered NO for INO therapy typically ranges from about 5-80 ppm, although some studies have reported benefits at levels less than 1 ppm. NO₂ concentrations of less than 1 ppm may also be of interest. The illustrated system 10 preferably monitors NO₂ in the range of about 0-10 ppm, NO in the range of 0-200 ppm and O₂ in the range of 0-100%. Appropriate alarms may be provided in connection with selected parameter limits.

As previously noted, the NO subsystem 18 receives parameter information from a variety of sensors associated with the respiratory circuit 12. The ventilator flow, pressure and temperature parameters apply primarily to the NO gas delivery function of the NO subsystem 18. In this regard, it will be appreciated that these ventilator parameters directly affect the relationship between the amount of NO containing gas delivered to the inspiratory line 28 and the concentration of NO delivered to the patient 14. The patient flow, pressure, temperature and measured NO₂, NO and O₂ concentrations apply primarily to the monitoring or feedback function of the NO subsystem 18.

It will be appreciated that the measured concentrations will represent a weighted average gas concentration at the patient interface due to the longer response time of the electrochemical sensor cells in relation to the patient circuit flow dynamics.

Accordingly, this relationship may be theoretically or empirically derived in order to increase system accuracy. In connection with the illustration system, this relationship has been empirically derived and may be continually or periodically updated based on experience with the system 10.

This empirical information is stored in controller 60 which may include a microprocessor based computing system. The controller 60, which controls operation of the NO subsystem 18 via a communications link such as an RS-232 interface, receives the respiratory circuit parameter information from the various sensors as described above. The controller 60 also receives inputs from a user interface such as a keypad, cursor device or touch screen (not shown) regarding patient treatment parameters such as desired NO delivery concentration. Based on this information, the controller 60 provides signals to control delivery of the NO containing gas. In response to these signals, the NO subsystem 18 delivers NO containing gas from the source 16 to the inspiratory limb 22 via NO₂ scrubber 62 which removes NO₂ from the gas. Fig. 1 also shows a conventional scavenge system 20 that scavenges the side stream gas prior to release to the ambient environment.

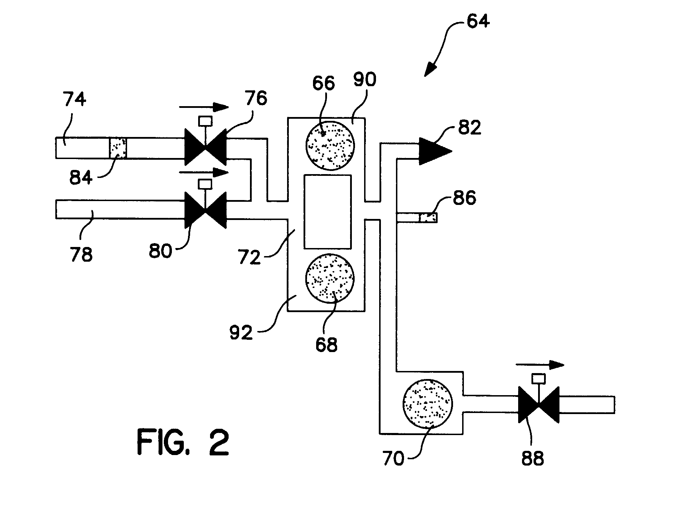

Fig. 2 illustrates one embodiment of a side stream gas monitoring assembly 64 that can be implemented as part of the NO subsystem 18 of Fig. 1. As noted above, NO gas injection and side stream gas measurement are monitored and controlled together to allow for more accurate administration of INO therapy. The gas monitoring assembly 64 of Fig. 2 provides direct side stream measurement information regarding NO, NO₂ and O₂ concentrations proximate to the patient. The illustrated assembly 64 includes NO₂ and NO electrochemical cells 66 and 68, respectively, in parallel flow lines, and an O₂ electrochemical cell 70 disposed downstream from the NO and NO₂ cells 66 and 68. Sample gas is delivered into a manifold section 72 of the assembly 64 via side stream flow line 74 and sample gas valve 76. Air may also be delivered to the manifold section 72 via vent line 78 and vent valve 80. The valves 76 and 80 allow gas to flow to the sensors 66, 68 and 70. As previously noted, the sample gas is saturated. The vent line 78 and valve 80 allows air to mix with the saturated gas so as to reduce the relative humidity of the gas to within the operating range of the cells 66, 68 and 70. The vent valve 80 can also be opened to auto zero the cells 66, 68 and 70 and pressure sensor 82. A temperature sensor84 is mounted in side stream flow line 74 to allow for temperature related concentration adjustments, thereby improving accuracy.

From the manifold section 72, the gas flows through parallel flow lines 90 and 92 containing cells 66 and 68. Alternatively, the cells 66 and 68 may be arranged in series in a single flow line. However, the parallel flow path structure reduces measurement interference. Electrochemical cells include an electrolyte solution designed to be reactive to the specific type of gas to be measured. The gas is exposed to the electrolyte solution through a diffusion barrier interface. The reaction of the gas with the electrolyte solution produces an electric current that is proportional to the amount of the specific gas to be measured in the gas sample. Although electrochemical cells are designed to be selective to the specific gas to be measured by the cell, NO cells consume NO₂ and vice versa. The parallel design reduces such sources of interference error.

A temperature sensor 86, pressure sensor 82 and the O₂ cell 70 are positioned downstream from the parallel flow section. Temperature sensor 86 is employed in conjunction with sensor 84 to allow for temperature related adjustments to the concentration measurement. Pressure sensor 82 is used in conjunction with valves 76 and 80 to ensure that the cells 66-70 receive adequate flow for the desired measurements. The O₂ cell can be located downstream as shown due to the reduced O₂ cell sensitivity. Gas consumption by the NO and NO₂ cells 66 and 68 will not significantly affect the O₂ cell measurement. Moreover, arranging the O₂ cell in series with the parallel flow section eases flow past the NO and NO₂ cells 66 and 68. From the O₂ cell 70, the gas flows through scavenger valve 88 to a scavenger (not shown) prior to release to the ambient environment.

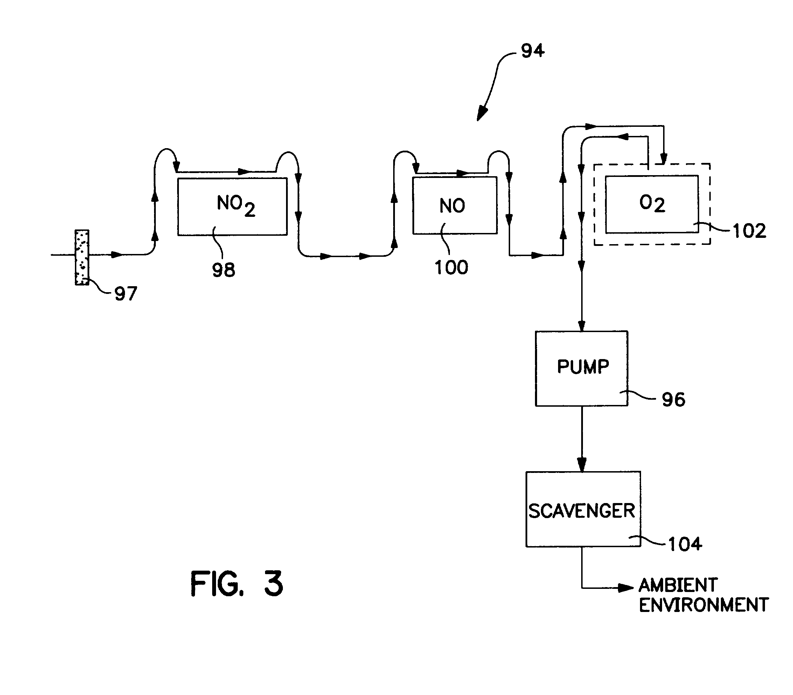

Fig. 3 is a pneumatic flow diagram showing an alternative embodiment of a gas monitoring assembly 94 according to the present invention. The sample gas is drawn through the assembly 94 by a pump 96 such as a conventional vacuum pump. As the gas passes through the assembly 94, it passes, in series, through hydrophobic filter 97 that removes liquid and other matter that may impair cell operation, across the diffusion barrier of an NO₂ electrochemical cell 98, across the diffusion barrier of an NO electrochemical cell 100 and around an O₂ electrochemical cell 102, all of which are located upstream from the pump 96. It is noted that the illustrated O₂ cell 102 works on a partial pressure principle, and is enveloped by the gas, rather than being exposed only at a diffusion barrier. Finally, the gas passes to scavenger 104 prior to release to the ambient environment.

Each of the cells 98, 100 or 102 is preferably enclosed in a sealed chamber so that the electrochemical reaction rates measured are due to the concentration of the gas to be measured, and is not due to pressure differences between the sample gas and the ambient environment. Because of this sealed environment and the positioning of the cells 98-102 upstream from the pump 96, the cells 98-102 will be maintained at a subambient pressure. Moreover, this cell positioning relative to the pump 96 facilitates isolation of the cells 98-102 from pressure fluctuations associated with pump operation. The illustrated sequence of the cells 98-102 with the NO₂ cell 98 located upstream from the NO cell 100 has been found to minimize measurement interference in the serial measurement environment.

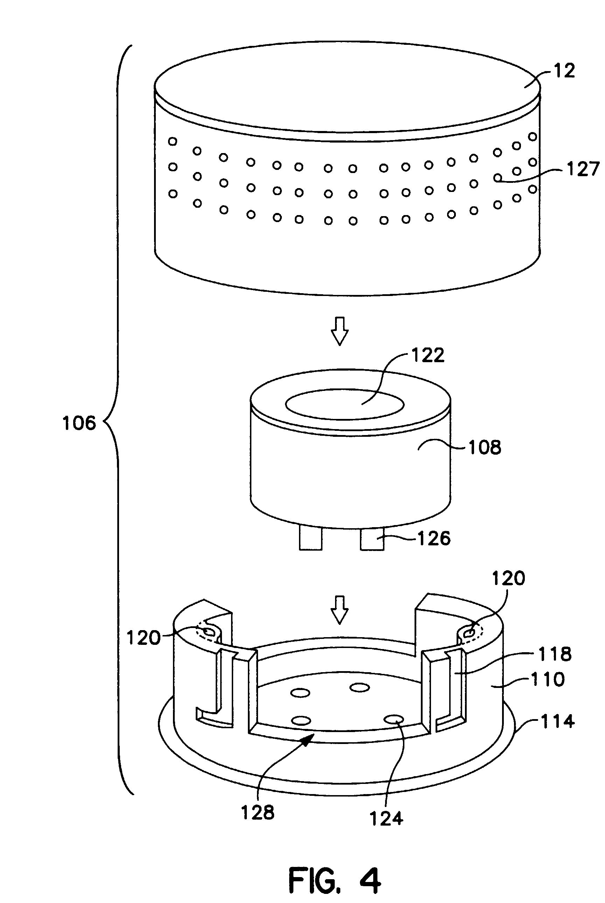

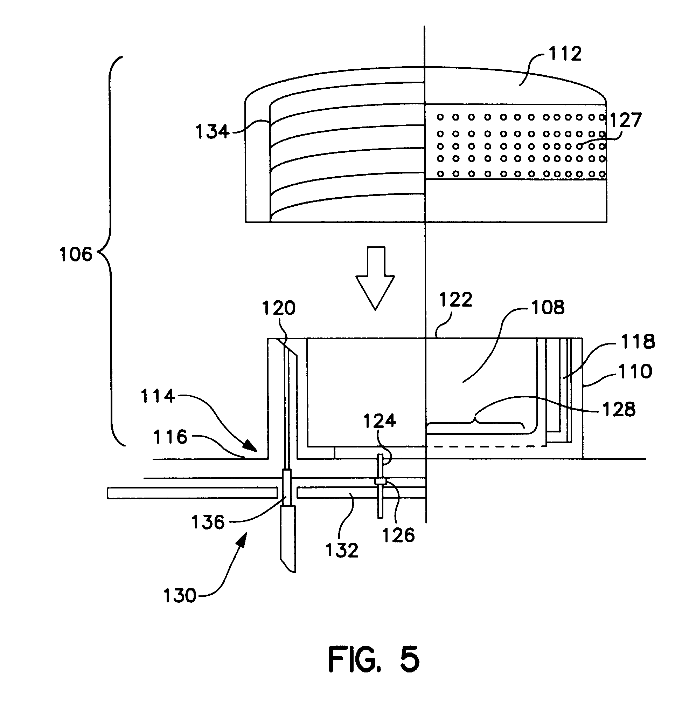

Figs. 4 and 5 show exploded, perspective and side, partially cross-section views, respectively, of an electrochemical cell housing 106 for an NO or NO₂ cell 108 according to the present invention. The housing 108, which may be constructed of molded plastic, includes a base 110 and a cover 112 that can be engaged to enclose the cell 108. An O-ring 114 seated in a groove 116 on the base 110 seals against the cover 112, when the cover 112 and base 110 are engaged, to seal the interior chamber where the cell 108 is housed. The cover 112 is maintained in the engaged position by a molded key lock channels 118 of the base 110 which receive corresponding molded lock tabs 134 (Fig. 5) formed on an inside surface of the cover 112. The base 110 also includes molded gas passageways 120 for routing sample gas across the cell diffusion barrier 122, and molded sockets 124 for receiving the terminal pins 126 of cell 108. Additionally, the base 110 includes an open area 128 to facilitate finger access to the cell 108. The illustrated cover 112 includes a finger grip 127 to facilitate engagement and disengagement.

The cross-section portion of Fig. 5 shows the mounting of the base 110 and cell 108 on a mount 130 including a printed circuit board (PCB) 132. As shown, the pins 126 extend through base sockets 124 when the cell 108 is plugged into the base 108 to electrically interface with the PCB 132. Pneumatic tubes 136 extend through the mount 130 and plug into the molded channels 118 of base 108 to deliver sample gas into and extract gas from the housing 106. The socket and pin pattern can be varied as between the NO₂ and NO cells to prevent misuse of the cells. In addition, the lock channel and tab configuration is arranged to ensure proper orientation of the cover 112 and alignment of any flow directing structure of the cover 112 with the gas passageways 120.

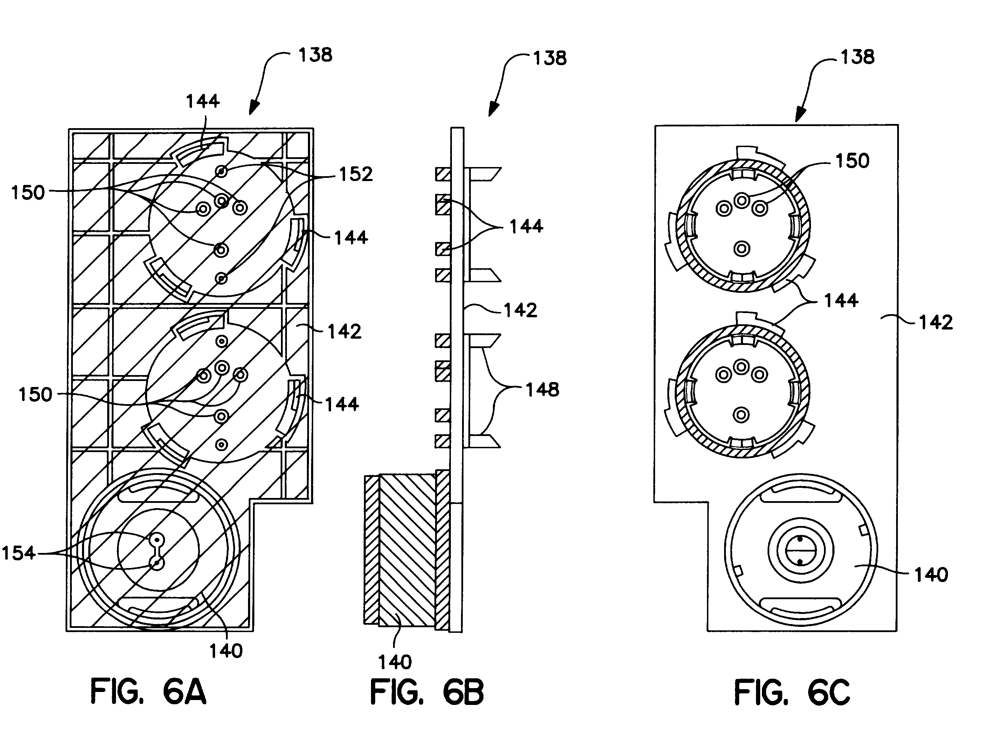

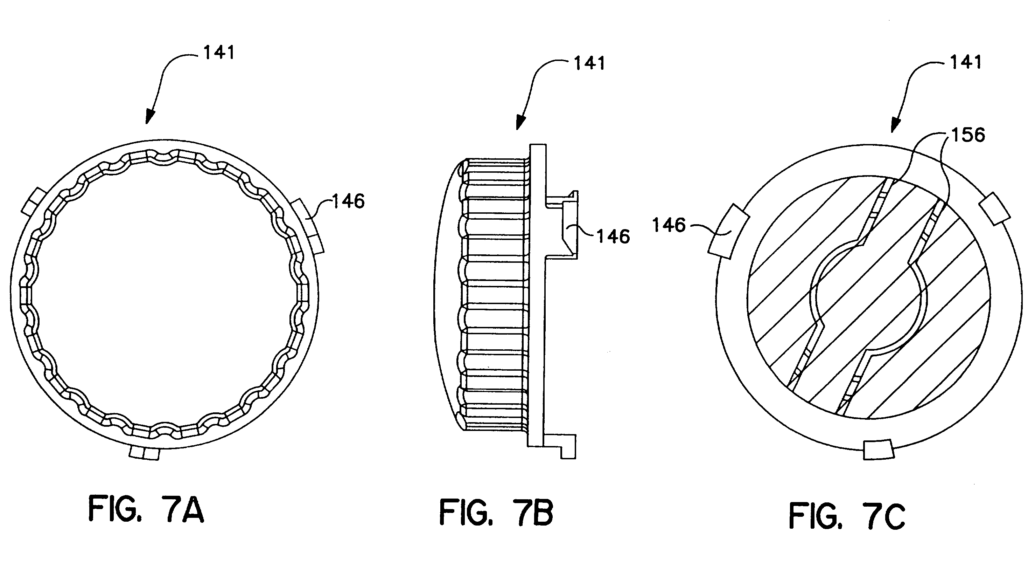

Figs. 6A - 7C show an alternative embodiment of a housing for the cells. More particularly, Figs. 6A - 6C show top, side and bottom views, respectively, of a housing base 138 with mounted O₂ cell cover 140. Figs. 7A - 7C show top, side and bottom views, respectively, of a cover 141 for the NO₂ or NO cell. Referring to Figs. 6A - 6C, the illustrated base 138, which may be constructed of molded plastic, includes a base plate 142, molded lock channel structures 144 extending upwardly from the base plate 142 to engage lock tab structures 146 (Figs. 7A - 7C) of the cover 141, registration pins 148 for use in mounting the base plate 142 in proper alignment with an underlying PCB (not shown), electrical sockets 150, and gas tube openings 152. Fig. 6A shows openings 154 on the upper surface of the O₂ cell cover 140 for delivering gas to and removing gas from the interior of the cover 140. Fig. 6C shows various structural elements for aligning and interfacing the base 138 with a PCB. Fig. 7C shows a rib structure 156 formed on the interior surface of the cover 141 for routing gas into a narrow channel between the openings 152 (Figs. 6A - 6C) over the electrochemical cell (not shown). Such routing promotes gas flow and reduces dead space, thereby improving cell response time. The invention, as described in the various exemplary embodiments above, thus allows for continuous and accurate INO therapy monitoring using electrochemical cell technology. In addition, the invention provides good sensor response times with reduced cross-sensor interference.

## Claims

1. A system for use in administering inhaled nitric oxide to a patient, comprising:
a supply of gas containing nitric oxide;
patient interface means for communicating with a respiratory system of said patient;
an inspiration flow line for delivering a first portion of said nitric oxide containing gas from said supply to said patient interface means;
a side stream flow line for receiving a second portion of said nitric oxide containing gas diverted from said inspiration flow line; and
an electrochemical cell, contained within a sealed housing associated with said side stream flow line for use in sensing an amount of nitric oxide in said diverted second gas portion, wherein said sealed housing maintains any pressure difference between an interior of said housing and an ambient environment.

2. A system as set forth in Claim 1, wherein said supply of gas comprises a gas canister containing nitric oxide and an inert gas.

3. A system as set forth in Claim 1, wherein said patient interface means comprises an intratracheal tube.

4. A system as set forth in Claim 1, wherein said side stream flow line comprises a gas passageway extending within a housing of said electrochemical cell.

5. A system as set forth in Claim 1, wherein said sealed housing comprises a first portion, a second portion and a seal at an interface between said first and second portions.

6. A system as set forth in Claim 1, further comprising a pump, wherein said pump is disposed downstream from said electrochemical cell relative to said side stream flow line.

7. A system as set forth in Claim 1, further comprising a nitrogen dioxide sensor, wherein said sensor is disposed downstream from said electrochemical cell relative to said side stream flow line.

8. A system as set forth in Claim 1, further comprising a nitrogen dioxide sensor, wherein said electrochemical cell and said nitrogen dioxide sensor are disposed in parallel flow lines.

9. A system as set forth in Claim 1, further comprising a nitrogen dioxide sensor and an oxygen sensor, wherein said electrochemical cell is disposed between the said nitrogen dioxide sensor and said oxygen sensor.

10. A system as set forth in Claim 1, further comprising a filter disposed between said supply and said electrochemical cell for removing undesired matter from said nitric oxide containing gas.

11. A system for use in administering inhaled nitric oxide to a patient, comprising:
a supply of gas containing nitric oxide;
a flow line for use in delivering at least a portion of said nitric oxide containing gas from said source;
pump means for drawing said gas from said supply into said flow line; and
a nitric oxide sensor pneumatically disposed between said supply and said pump.

12. A system as set forth in Claim 11, wherein said flow line includes an inspiration conduit portion, wherein said nitric oxide containing gas mixes with patient respiration gases.

13. A system as set forth in Claim 11, wherein said nitric oxide sensor comprises an electrochemical cell.

14. A system as set forth in Claim 11, further comprising a nitrogen dioxide sensor disposed between said supply and said nitric oxide sensor.

15. A system as set forth in Claim 11, further comprising a housing for sealingly enclosing said nitric oxide sensor.
